(19) Europäisches Patentamt
European Patent Office
Office européen des brevets

(11) Veröffentlichungsnummer: **0 003 313**
**B1**

(12) **EUROPÄISCHE PATENTSCHRIFT**

(21) Anmeldenummer: **79100127.4**

(22) Anmeldetag: **17.01.79**

(51) Int. Cl.³: **C 07 D 213/64,**
A 01 N 43/40

(54) **Neue Pyridyloxy-phenoxy-alpha-propionsäure-aminoalkyl-ester mit herbizider Wirkung, ihre Herstellung, sie enthaltende Mittel und deren Verwendung**

(30) Priorität: **27.01.78 CH 928/78**

(43) Veröffentlichungstag der Anmeldung:
**08.08.79 Patentblatt 79/16**

(45) Bekanntmachung des Hinweises auf die Patenterteilung:
**07.01.81 Patentblatt 81/01**

(84) Benannte Vertragsstaaten:
**BE CH DE FR GB IT NL**

(56) Entgegenhaltungen:
**DE-A-2 715 284**
**FR-2 288 089**
**FR-A-2 291 750**
**FR-A-2 359 129**

(73) Patentinhaber: **CIBA-GEIGY AG**
**Patentabteilung Postfach**
**CH-4002 Basel (CH)**

(72) Erfinder: **Schurter, Rolf, Dr.**
**Holzmattstrasse 45**
**CH-4102 Binningen (CH)**
**Rempfler, Hermann, Dr.**
**Allschwilerweg 67**
**CH-4102 Binningen (CH)**
**Böhner, Beat, Dr.**
**Hügelweg 3**
**CH-4102 Binningen (CH)**

**EP 0 003 313 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Neue Pyridyloxy-phenoxy-α-propionsäure-aminoalkyl-ester mit herbizider Wirkung, ihre Herstellung, sie enthaltende Mittel und deren Verwendung

Die vorliegende Erfindung betrifft neue Pyridyloxy-phenoxy-α-propionsäure-aminoalkyl-ester, welche herbizide und das Pflanzenwachstum regulierende Wirkung haben, Verfahren zu deren Herstellung, Mittel welche sie als Wirkstoffe enthalten sowie die Verwendung dieser Ester oder sie enthaltender Mittel als Herbizide oder zu Regulierung des Pflanzenwachstums.

Die Pyridyloxy-phenoxy-propionsäure-aminoalkyl-ester sind neue Verbindungen und entsprechen der Formel:

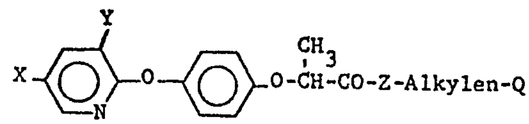

(I)

worin

X ein Halogenatom oder die Trifluormethylgruppe,

Y Wasserstoff, ein Halogenatom oder die Trifluormethylgruppe,

Z Sauerstoff oder Schwefel,

"Alkylen" einen unverzweigten oder verzweigten, gegebenenfalls durch Sauerstoff unterbrochenen $C_2$—$C_6$ Alkylrest,

Q ein Amino- oder Ammonio-Rest

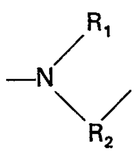 , 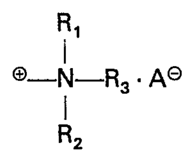 oder 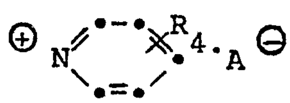

$R_1$ Wasserstoff, $C_1$—$C_6$ *Alkyl* unsubstituiert oder substituiert durch Hydroxyl, Halogen, Cyano, $C_1$—$C_4$ Alkoxy, Carboxyl oder $C_1$—$C_4$ Alkoxycarbonyl, $C_3$—$C_6$ *Alkenyl,* gegebenenfalls durch Halogen substituiert, $C_3$—$C_6$ *Alkinyl,*

$R_2$ dasselbe wie $R_1$ oder *Phenyl,* unsubstituiert oder durch Halogen, $C_1$—$C_4$ Alkyl, $C_1$—$C_4$ Alkoxy, $C_1$—$C_4$ Alkylthio, Cyano, Nitro oder Trifluoromethyl substituiert,

$R_1$ $R_2$ zusammen mit dem Stickstoffatom an das sie gebunden sind, ebenfalls einen 5—6 gliedrigen Heterocyclus, der als zweites Heteroatom Sauerstoff, Schwefel oder Stickstoff enthalten und durch $C_1$—$C_4$ Alkyl oder Phenyl substituiert sein kann,

$R_3$ dasselebe wie $R_1$

$R_4$ Wasserstoff, Methyl, Aethyl oder Halogen und

$A^{\ominus}$ das Anion einer organischen oder anorganischen Säure

bedeuten.

In dieser Formel sind die Halogenatome vorzugsweise Chlor oder Brom. Die Alkyl- oder Alkenylreste enthalten die angegebene Anzahl Kohlenstoffatome und können verzweigt oder unverzweigt sein. Als Alkenylrest sind der gegebenenfalls chlorierte Allyl- und Methallylrest bevorzugt, der bevorzugte Alkinylrest ist de Propargylrest. Die 5—6 gliedrigen Heterocyclen Q, welche die Reste $R_1$ und $R_2$ zusammen mit dem Stickstoffatom, an das sie gebunden sind bilden, kommen vorzugsweise die Pyrrol-, Pyrrolidin-, Pyridin-, Piperidin-, Morpholin-, Thiomorpholin- sowie der Piperazin-Ring in Frage. Diese Ringe können durch einen $C_1$—$C_4$ Alkylrest, vorzugsweise Methyl substituiert sein, der Piperazinrest ebenfalls durch Phenyl.

Aus neuen Veröffentlichungen sind Phenoxy-phenoxy-propionsäureester und Pyridyloxy-phenoxy-propionsäureester mit ähnlicher chemischer Struktur bekannt geworden, siehe die DOS 2 546 251, 2 617 804, 2 623 558.

Die erfindungsgemässen Wirkstoffe der Formel I und die sie als aktive Komponente enthaltenden herbiziden Mittel sind insbesondere brauchbar zur selektiven Bekämpfung von schwer bekämpfbaren Ungräsern in Kulturpflanzenbeständen, auch in monokotylen Kulturpflanzen, wie Weizen und anderen Getreidesorten. Die erfindungsgemässen Verbindungen sind also gegenüber Kulturpflanzen wie Weizen, gut verträglich und gegen Ungräser sehr wirksam.

Die bereits bekannten durch die Amino- oder Ammonio-Reste in der Esterseitenkette substituierten Phenoxy-phenoxy-alkancarbonsäureester des Standes der Technik, wie die bereits erwähnten Ester und Thioester der DT—OS 2 617 804 resp. 2 623 558 sind gegenüber empfindlichen Kulturpflanzen wie z.B. weizen entweder zu agressiv oder zeigen bei guter Verträglichkeit eine zu geringe Aktivät gegenüber den zu bekämpfenden Ungräsern.

Die erfindungsgemässen Wirkstoffe zeichnen sich dem gegenüber durch eine deutlich bessere Verträglichkeit gegenüber Kulturpflanzen, wie insbesondere Weizen aus (Selektivität) oder durch eine bessere Aktivität gegen Ungräser, wie insbesondere Avena fatua (Wildhafer)aus.

Aufgabe dieser Erfindung war also, neue Derivate der Reihe der 4-(Pyridyloxy)-$\alpha$-phenoxy-propion- und -propionthiolsäuren zu schaffen, welche bekannten Verbindungen ähnlicher Struktur in der herbiziden Wirkung gegen schwer bekämpfbare Ungräser überlegen und gegenüber wichtigen Kulturpflanzen wie Weizen verträglicher sind, also eine bereicherung der Technik darstellen.

Zur Herstellung der neuen Ester der Formel I dienen an sich bekannte Verfhren:

Nach einem dieser Verfahren setzt man ein entsprechendes 4 - (3',5' - Dihalogen - pyridyl - (2') - oxy) - $\alpha$ - phenoxy - propionsäurehalogenid der Formel II

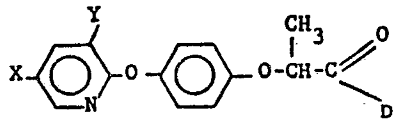

(II)

worin X und Y die unter Formel I gegebene Bedeutung haben und "D" ein Halogenatom oder einen reaktionsfähigen Esterrest darstellt, in Gegenwart eines basischen Säureakzeptors, mit einem Alkohol oder Thiol der Formel III

H—Z-Alkylen-Q (III)

um, worin "Alkylen", Q und Z die unter Formel I gegebene Bedeutung haben.

Gemäss einem weiteren Verfahren setzt man den entsprechenden Hydroxy-phenyl-pyridyläther oder ein Salz desselben, von der Formel IV

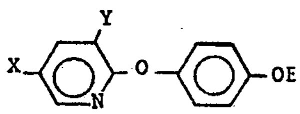

(IV)

worin
X und Y die unter formel I gegebene Bedeutung haben und E Wasserstoff oder das Aequivalent eines alkalimetall- oder Erdalkalimetallkations bedeutet, mit einem $\alpha$-Halogenpropion(thiol)säureester der Formel V

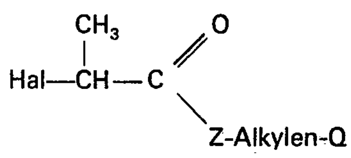

(V)

in Gegenwart eines säurebindenden Mittels (Base) um. In diesen Formeln haben "Alkylen", Hal, Q und Z die unter Formel I gegebene Bedeutung.

Schliesslich lassen sich die Verbindungen der Formel I, auch in bekannter Weise herstellen, indem man eine Verbindung der Formel VI

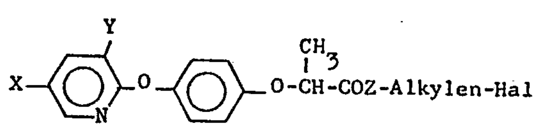

(VI)

worin "Alkylen" X, Y und Z die unter Formel I gegebene Bedeutung haben, während "Hal" ein Halogenatom bedeutet, mit einem Amin der Formel VII

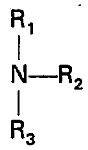

(VII)

worin $R_1$, $R_2$ und $R_3$ die unter Formel I, gegebene Bedeutung haben umsetzt.

Quaternäre Ammoniumverbindungen können ferner auch hergestellt werden, indem man eine Verbindung der Formel I, in der Q den Aminorest

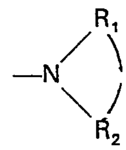

bedeutet, mit einer Verbindung der Formel VIII

$$A—R_3 \qquad (VIII)$$

in üblicher Weise umsetzt. In diesen Formeln haben $R_1$, $R_2$, $R_3$ und A die unter Formel I gegebene Bedeutung.

Diese Umsetzungen werden in Lösungsmitteln bei Normaldruck oder im Druckgefäss bei 0—180°C vorzugsweise bei 20—150°C vorgenommen. Zur Beschleunigung der Reaktion kann das Reaktionsgemisch bis zum Siedepunkt des Lösungsmittels erwärmt werden.

Die Umsetzungen werden vorzugsweise in einem gegenüber den Reaktionskomponenten inerten Lösungsmittel durchgeführt. Als Lösungsmittel kommen solche aus den verschiedensten Stoffklassen in Frage, wie aliphatische und aromatische, gegebenenfalls chlorierte Kohlenwasserstoffe, z.B. Aethylenchlorid etc., sowie polare organische Lösungsmittel, wie Alkohole, Aether, Ketone, Amide, stabile Ester, z.B. Methyläthylketon, Dimethoxyäthan, Dimethylformamid, Dimethylsulfoxid, Tetrahydrofuran etc.

Als basische Säureakzeptoren für die Umsetzung mit den Halogenverbindungen der Formel II und V können wässerige Alkalimetallhydroxide, wie KOH und NaOH sowie weiter übliche basische Stoffe, wie Karbonate ($K_2CO_3$, $NaHCO_3$), Alkoholate ($NaOCH_3$ und Kalium-tert. butylat), aber auch organische Basen wie Triäthylamin etc. verwendet werden.

Die Ausgangsstoffe der Formeln II bis V sind zum Teil bekannt oder können nach bekannten Verfahren hergestellt werden.

Zu den Propionthiolsäureestern der Formel I (Z = Schwefel) kann man auch gelangen, indem man die entsprechende freie 4-(Pyridyl-(2′)-oxy)-α-phenoxy-propionthiolsäure bezw. ein Metallsalz dieser Säure mit einem Aminoalkyl-Halogenid der Formel Hal-Alkylen-Q in Gegenwart einer Base umsetzt.

Die oben genannte freie Propionthiolsäure und ihre Metallsalze sowie deren herstellung aus dem entsprechenden Propionsäurehalogenid mit Schwefelwasserstoff, $Na_2S$ oder NaHS in Gegenwart eines basischen Säureakzeptors ist Gegenstand einer hängigen Patentanmeldung. Die nach dieser Methode hergestellte 4-(3′,5′-Dichlor-pyridyl-(2′)-oxy)-α-phenoxy-propionthiolsäure ist ein Oel vom Brechungsindex $n_D^{21} = 1{,}5787$, das nach Kristallisation bei 85—87°C schmilzt.

Die nachfolgenden Beispiele veranschaulichen die Herstellung einiger erfindungsgemässer Wirkstoffe der Formel I. Weitere in entsprechender Weise oder nach einer anderen der im Text erwähnten Methoden hergestellte Endstoffe der Formel I sind anschliessendtabellarisch aufgeführt.

### Beispiel 1.

α - [4 - (3′,5′ - Dichlorpyridyl - 2′ - oxy) - phenoxy] - propionsäure - dimethylaminoäthylester

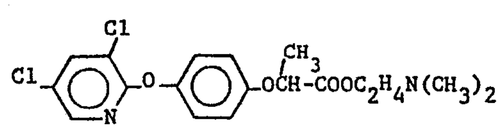

a) 51,9 (0,15 Mol) α-[4-(3′,5′-Dichlorpyridyl-2′-oxy)-phenoxy]-propionsäurechlorid werden bei Raumtemperatur zu einem Gemisch von 15,0 g (0,165 Mol) 2-Dimethylaminoäthanol, 22,9 ml (0,165 Mol) Triäthylamin und 300 ml Methylenchlorid getropft. Dabei lässt man die Temperatur auf 35° steigen. Nach 1 Std. ausrühren gibt man 100 ml Wasser zu und rührt gut durch. Die organische Phase wird direkt über kleine Kieselgelsäule filtriert. Beim Eindampfen des Filtrats erhält man 55,3 g (92.3% der Theorie) des obigen Produktes als gelbes klares Oel mit dem Brechungsindex $n_D^{20}$ 1,5498.

Das als Ausgangsmaterial benötigte Säurechlorid wird wie folgt hergestellt:

b) 26.8 g (0,082 Mol) α-(4-(3′,5′-Dichlorpyridyl-2′-Oxy)-phenoxy)-propionsäure werden mit 30 ml Thionylchlorid versetzt und, nachdem die Gasentwicklung abgeklungen ist, auf 50°C erwärmt. Nach 2 Std. wird das Reaktions-gemisch am Vacuum eingedampft, mit 100 ml Toluol versetzt und wieder eingedampft. Als Produkt erhält man ein dunkelbraunes Oel, das langsam zu kristallisieren beginnt. Es werden 25,9 g (86,7%) α-(4-(3′,5′-Dichlorpyridyl-2′-oxy)-phenoxy)-propionsäurechlorid vom Schmelzpunkt 45°C erhalten.

Beispiel 2.

$\alpha$ - [4 - (3',5' - Dichlorpyridyl - 2' - oxy) - phenoxy] - thiopropionsäure - S - diäthylamino - äthylester

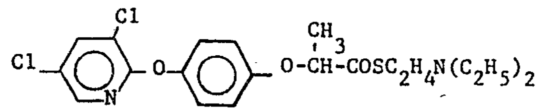

a) 16,0 g (0,046 Mol) $\alpha$-[4-(3',5'-Dichlorpyridyl-2'-oxy)-phenoxy]-thiopropionsäure werden in 50 ml Methyläthylketon gelöst vorgelegt und bei Raumtemperatur mit 14,4 g (0,104 Mol) Kaliumcarbonat versetzt. Durch die exotherme Reaktion steigt die Temperatur auf 28°C. Nach 30 Min. gibt man 8,6 g (0,05 Mol) 2-Diäthylaminoäthylchlorid·HCl zu, wobei die Temperatur rasch auf 40° steigt, 1 Std. bei dieser Temperatur ausrühren lassen. Dann wird das Reaktionsgemisch direkt über eine kleine Kieselgelsäule abfiltriert. beim Eindampfen des Filtrates erhält man ein gelbes, klares Oel, 8,6 g )42,2% de Theorie) des obigen Produktes das den Brechnungsindex $n_D^{20}$ 1,5616 hat.

Die als Ausgangsmaterial benutzte Thiopropionsäure wurde wie folgt hergestellt:

17,2 g (0,0496 Mol) $\alpha$-(4-(3',5'-Dichlorpyridyl-2'-oxy)-phenoxy)-propionsäurechlorid, hergestellt gemäss Beispiel 1b.

werden einem Gemisch von 8,9 g KOH in 4,9 ml Wasser und 75 ml Dimethoxyäthan zugetropft, das bei 10—15°C unter starkem Rühren mit $H_2S$ gesättigt wurde. Während des Zutropfens hält man die Temperatur mit einem Eisbad bei 10°C. Anschliessend lässt man 30 Min. bei Raumtemperatur ausrühren und giesst das Reaktionsgemisch auf 150 ml Eis/Wasser. Die trübe, braune Lösung wird mit conc. HCl auf pH 1 gestellt. Dabei fällt ein braunes Oel aus, das in Methylenchlorid aufgenommen wird. Die organische Phase wird direkt auf eine kleine Kieselgelsäule gegeben und mit Methylenchlorid durchgespült. Nach dem Eindampfen der hellgelben Lösung erhält man ein oranges, klares Oel. Dieses kristallisiert beim Verreiben mit Petroläther. Man erhält 16,8 g $\alpha$-(4-(3',5'-Dichlorpyridyl-2'-oxy)-phenoxy)-propionthiolsäure als gelbe Kristalle vom Schemzpunkt 85—87°C.

Beispiel 3.

$\alpha$ - [4 - (3'.5' - Dichlorpyridyl - 2' - oxy) - phenoxy] - thiopropionsäure - S - (2 - diäthylmethyl - ammonium) - äthylester - iodid

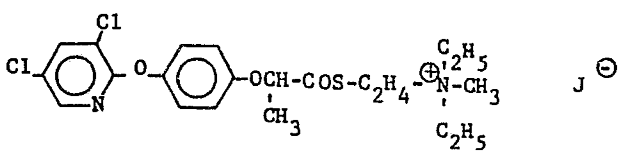

4,6 g (0,0104 Mol) des gemäss Beispiel 2 erhaltenen $\alpha$ - [4 - (3',5' - Dichlorpyridyl - 2' - oxy) - phenoxy)] - thiopropionsäure - S - (2 - diäthylamino) - äthylester werden bei Raumtemperatur mit 20 ml methyljodid versetzt. Das Reaktionsgemisch wird für 10 Min. auf 35°C erwärmt. Dann wird es am Vacuum eingeengt, wobei ein fester Rückstand bleibt. Diesen zerreibt man mit ein wenig Aether, filtriert ab und erhält nach dem Trocknen 4,2 g (70% de Theorie) des obigen Produktes als gelbes kristallines Pulver, das bei 80° schmilzt.

Beispiel 4.

$\alpha$ - [4 - (3',5' - Dichlorpyrid - 2' - yloxy) - phenoxyl] - propionsäure - 2 - N - piperidinoäthyl - ester

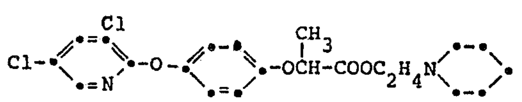

a) 15,2 (0,035 M) $\alpha$ - [4 - (3',5' - Dichlorpyridyl - 2' - oxy) - phenoxy] - propionsäure - 2 - bromäthylester werden zusammen mit 10 ml Toluol und 7,4 ml (0,075 M) Piperidin auf 100°geheizt. Dabei geht alles in Lösung und das Dünnschichtchromatogramm zeigt nach 2 Stunden kein Ausgangsmaterial mehr an. Das Reaktionsgemisch wird mit wenig Essigester verdünnt, und mit Wasser gewaschen. Die organische Phase wird mit Natriumsulfat getrocknet und eingedampt. Dabei erhält man 13,2 g (85,7%) Titelprodukt mit dem Brechungsindex $n_D^{21}$ 1.5448.

Das Ausgangsprodukt wurde wie folgt erhalten:

b) 173,0 g (0,5 M) $\alpha$ - [4 - (3',5' - Dichlorpyridyl - 2' - oxy) - phenoxy) - propionsäurechlorid werden bei 10° zu einem Gemisch von 39 ml (0,55 M) Aethylenbromhydrin, 76 ml (0,55 M) Triäthylamin in 300 ml Toluol zugetropft. Die Reaktion verläuft leicht exotherm. Nach 10 Minuten ausrühren findet man im Dünnschichtchromatogramm kein Ausgangsmaterial mehr. Das Reaktionsgemisch wird mit wenig Wasser gewaschen. Die organische Phase trocknet man mit Natriumsulfat und dampft sie ein. Als Rückstand erhält man ein gelbes, klares Oel, das beim Zerreiben mit Petroläther weiss kristallisiert und 181 g (83,4%) $\alpha$ - (4 - (3',5' - Dichlorpyridyl - 2' - oxy) - phenoxy) - propionsäure - 2 - bromäthylester mit dem Smp. 106—7° ergibt.

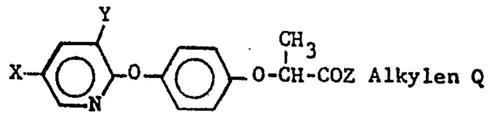

| No. | X | Y | −Z−Alkylen Q | physikalische Konstante |
|---|---|---|---|---|
| 1 | Cl | Cl | $-O-C_2H_4N(CH_3)_2$ | $n_D^{20}$ 1.5498 (Beispiel 1) |
| 2 | Cl | Cl | $-S-C_2H_4N(C_2H_5)_2$ | $n_D^{20}$ 1.5616 (Beispiel 2) |
| 3 | Cl | Cl | $-S-C_2H_4\overset{CH_3}{\overset{\mid}{N}}{}^{\oplus}\ (C_2H_5)_2\ I^{\ominus}$ | Smp. 80° (Beispiel 3) |
| 4 | Cl | H | $-OC_3H_6N(CH_3)_2$ | |
| 5 | Cl | CN | $-OC_2H_4N(CH_3)_2$ | |
| 6 | Br | Br | $-OC_2H_4N^{\oplus}\ (CH_3)_3\ Br^{\ominus}$ | |
| 7 | Br | H | $-S-C_2H_4N(C_2H_5)_2$ | |
| 8 | $CF_3$ | H | $-OC_2H_4N(CH_3)_2$ | |
| 9 | $CF_3$ | Cl | $-OC_3H_6NHCH_3$ | |
| 10 | $CF_3$ | H | $-S-C_2H_4-$ 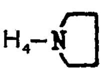 | |
| 11 | Cl | Cl | $-O-C_2H_4-$ 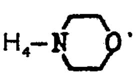 | $n_D^{22}$ 1.5652 |
| 12 | Cl | Cl | $-S-C_2H_4-$ 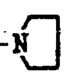 | |
| 13 | Cl | CN | $-S-C_3H_6-\overset{CH_3}{\overset{\mid}{N}}-CH_2-CH=CH_2$ | |
| 14 | Cl | Cl | $-O-C_2H_4N(CH_2-CH=CH_2)_2$ | $n_D^{22}$ 1.5586 |
| 15 | Cl | H | $-OC_2H_4N(C_2H_4OH)_2$ | |
| 16 | Cl | H | $-OC_2H_4\overset{CH_3}{\overset{\mid}{N}}{}^{\oplus}\ (C_2H_4OH)_2\ Br^{\ominus}$ | |

| No. | X | Y | –Z–Alkylen Q | physikalische Konstante |
|---|---|---|---|---|
| 17 | Cl | Cl | $-OC_2H_4-\overset{\oplus}{N}(CH_3)_2-CH_2-CH=CH_2\ Cl^{\ominus}$ | Smp. 80° (Zers.) |
| 18 | Cl | Cl | 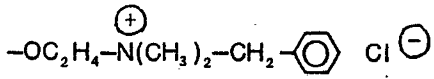 | Smp. 75° (Zers.) |
| 19 | Cl | Cl | 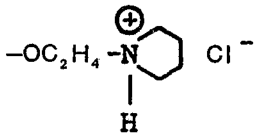 | Smp. 162–64° |
| 20 | Cl | Cl | 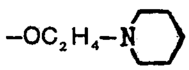 | $n_D^{21}$ 1.5448 (Beispiel 4) |
| 21 | Cl | Cl | 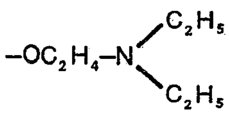 | $n_D^{21}$ 1.5415 |
| 22 | Cl | Cl | 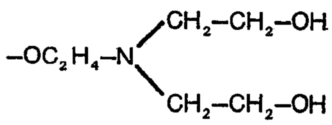 | $n_D^{21}$ 1.5598 |
| 23 | Cl | Cl | 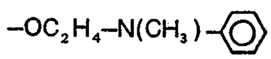 | $n_D^{21}$ 1.5781 |
| 24 | Cl | Cl | $-OC_2H_4-N(CH_3)-CH(CH_3)_2$ | $n_D^{21}$ 1.5501 |
| 25 | Cl | Cl | 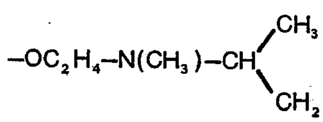 | $n_D^{21}$ 1.5572 |
| 26 | Cl | Cl | 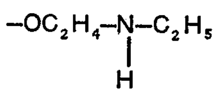 | $n_D^{21}$ 1.5511 |
| 27 | Cl | Cl | 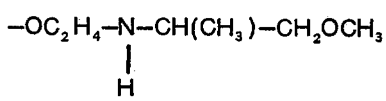 | $n_D^{21}$ 1.5397 |
| 28 | Cl | Cl | 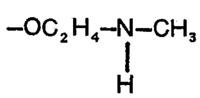 | $n_D^{21}$ 1.5543 |
| 29 | Cl | Cl | 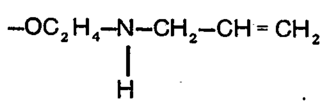 | $n_D^{21}$ 1.5578 |
| 30 | Cl | Cl | 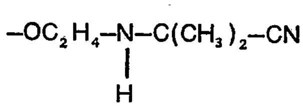 | |
| 31 | Cl | Cl | 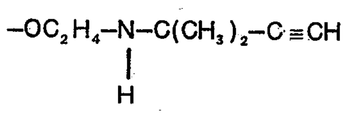 | $n_D^{21}$ 1.5507 |
| 32 | Cl | Cl | $-OC_2H_4-N(CH_3)-CH_2-COOC_2H_5$ | |

| No. | X | Y | –Z–Alkylen Q | physikalische Konstante |
|---|---|---|---|---|
| 22 | Cl | Cl | 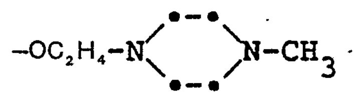 | $n_D^{21}$ 1.5580 |
| 34 | Cl | Cl | 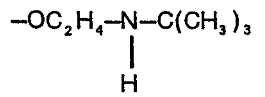 | $n_D^{21}$ 1.5433 |
| 35 | Cl | Cl | 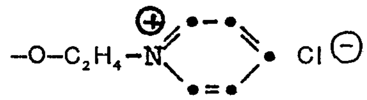 | $n_D^{21}$ 1.5930 |
| 36 | Cl | Cl | 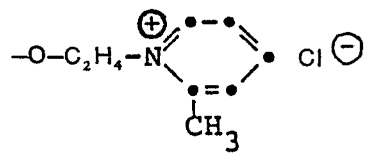 | |
| 37 | Cl | Cl | 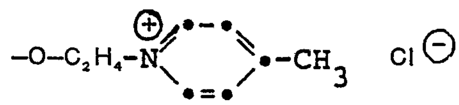 | |
| 38 | Cl | Cl | 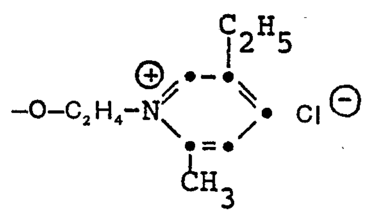 | |

Die Erfindung betrifft auch herbizide Mittel, welche einen neuen Wirkstoff der Formel I enthalten, sowie Verfahren zur pre-und insbesondere post-emergenten Unkrautbekämpfung, insbesondere von monocotylen Ungräsern.

Die erfindungsgemässen Mittel können in den üblichen Formulierungen vorliegen.

Die Herstellung erfindungsgemässer Mittel erfolgt in an sich bekannter Weise durch inniges Vermischen und Vermahlen von Wirkstoffen der Formel I mit geeigneten Trägerstoffen, gegebenenfalls unter Zusatz von gegenüber den Wirkstoffen inerten Dispersions- oder Lösungsmitteln. Die Wirkstoffe können in den folgenden Aufarbeitungsformen vorliegen und angewendet werden:

feste Aufarbeitungsformen:
Stäubemittel, Streumittel, Granulate, Umhüllungsgranulate Imprägnierungsgranulate und Homogengranulate;
in Wasser dispergierbare Wirkstoffkonzentrate:
Spritzpulver, (wettable powder), Pasten, Emulsionen;
flüssige Aufarbeitungsformen:
Lösungen.

Die Herstellung erfindungsgemässer Mittel wird durch die folgenden Formulierungsbeispiele veranschaulicht.

Spritzpulver

Zur Herstellung eines a) 50%igen, b) 25%igen und c) 10%igen Spritzpulvers werden folgende Bestandteile verwendet:

a) 50 Teile α - [4 - (3',5' - Dichlor - pyridyl - 2' - oxy) - phenoxy] - propionsäure - dimethylamino - äthyl - ester,
5 Teile Natriumdibutylnaphthylsulfonat,
3 Teile Naphthalinsulfonsäuren - Phenolsulfonsäuren - Formaldheyd - Kondensat 3:2:1,
20 Teile Kaolin,
22 Teile Champagne-Kreide;

b) 25 Teile des obigen Wirkstoffs,
5 Teile Oleylmethyltaurid-Natrium-Salz,
2,5 Teile Naphthalinsulfonsäuren-Formaldehyd-Kondensat,
0,5 Teile Carboxymethylcellulose,
5 Teile neutrales Kalium-Aluminium-Silikat,
62 Teile Kaolin;

c) 10 Teile des obigen Wirkstoffs,
3 Teile Gemisch der Natriumsalze von gesättigten Fettalkoholen,
5 Teile Naphthalinsulfonsäuren-Formaldehyd-Kondensat,
82 Teile Kaolin.

Der angegebene Wirkstoff wird auf die entsprechenden Trägerstoff (Kaolin und Kreide) aufgezogen und anschliessend vermischt und vermahlen. Man erhält Spritzpulver von vorzüglicher Benetzbarkeit und Schwebefähigkeit. Aus solchen Spritzpulvern können durch Verdünnen mit Wasser Suspensionen jeder gewünschten Wirkstoffkonzentration erhalten werden. Derartige Suspensionen werden zur Bekämpfung von Unkräutern und Ungräsern in Kulturpflanzungen im Vorauflaufverfahren und zur Behandlung von Rasenanlagen verwendet.

Paste

Zur Herstellung einer 45%igen Paste werden folgende Stoffe verwendet:

45 Teile α - [4 - (3',5' - Dichlorpyridyl - 2' - oxy) - phenoxy] - propionthiolsäure - S - diäthylamino - äthyl - ester,
5 Teile Natriumaluminiumsilikat,
14 Teile Cetylpolyäthylenglykoläther mit 8 Mol Aethylenoxid,
1 Teil Oleylpolyäthylenglykoläther mit 5 Mol Aethylenoxid,
2 Teile Spindelöl,
23 Teile Wasser,
10 Teile Polyäthylengylkol.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Emulsionskonzentrat

Zur Herstellung eines 25%igen Emulsionskonzentrates werden

25 Teile α - [4 - (3',5' - Dichlorpyridyl - 2' - oxy) - phenoxy] - propionthiolsäure - 5 - diallylaminoäthyl - ester,
10 Teile Mischung von Nonylphenolpolyoxyäthylen und Calcium-dodecylbenzol-sulfonat,
10 Teile Cyclohexanon,
55 Teile Xylol,

miteinander vermischt. Dieses Konzentrat kann mit Wasser zu Emulsionen auf zur Anwendung geeignete Konzentrationen verdünnt werden.

Anstatt des jeweiligen in den vorhergehenden Formulierungsbeispielen angegebenen Wirkstoffs kann auch eine andere der von der Formel I umfassten Verbindungen verwendet werden.

Erfindungsgemässe Mittel, die als aktive Komponente mindestens eine Verbindung der Formel I enthalten, eignen sich besonders zur selektiven Bekämpfung schwerbekämpfbarer monocotyler Ungräser in pre- und insbesondere post-emergenter Anwendung in Kulturpflanzenbeständen, wie z.B. Weizen, aber auch Soja, Baumwolle, Zuckerrohr etc.

Zum Nachweis der Brauchbarkeit als Herbizide (pre- und post-emergent) dienen folgende Testmethoden:

Pre-emergente Herbizid-Wirkung (Keimhemmung)

Im Gewächshaus wird unmittelbar nach der Einsaat der Versuchspflanzen in Saatschalen die Erdoberfläche mit einer wässerigen Dispersion der Wirkstoffe, erhalten aus einem 25%igen Emulsionskonzentrat resp. aus einem 25%igen Spritzpulver mit Wirkstoffen, die wegen ungenügender Löslichkeit nicht als Emulsionskonzentrat hergestellt werden können, behandelt. Es wurden vier verschiedene

Konzentrationsreihen angewendet, entsprechend 4, 2, 1 und 0,5 kg Wirksubstanz pro Hektar. Die Saatschalen werden im Gewächshaus bei 22—25°C und 50—70% rel. Luftfeuchtigkeit gehalten und der Versuch nach 3 Wochen ausgewertet und die Resultate nach folgender Notenskala bonitiert:

1 = Pflanzen nicht gekeimt oder total abgestorben
2—3 = sehr starke Wirkung
4—6 = mittlere Wirkung
7—8 = geringe Wirkung
9 = keine Wirkung (wie unbehandelte Kontrolle)
— = Pflanze in dieser Wirkstoffkonzentration nicht geprüft.

als Versuchspflanzen dienen zum Beispiel:

| | |
|---|---|
| hordeum (Gerste) | setaria italica |
| triticum (Weizen) | echinochloa crus galli |
| zea (Mais | beta vulgaris |
| sorghum hybr. (Hirse) | sida spinosa |
| oryza (Reis) | sesbania exaltata |
| glycine (Soja) | amaranthus retroflexus |
| gossypium (Baumwolle) | sinapis alba |
| avena fatua | ipomoea purpurea |
| lolium perenne | galium aparine |
| alopecurus myosuroides | pastinaca sativa |
| bromus tectorum | rumex sp. |
| cyperus esculentus | chrysanthemum leucum. |
| rottboellia exaltata | abutilon sp. |
| digitaria sanguinalis | solanum nigrum |

Post-emergente Herbizid-Wirkung (Kontaktherbizid)

Eine grössere Anzahl (mindestens 7) Unkräuter und Kulturpflanzen, sowohl monocotyle wie dicotyle, wurde nach dem Auflaufen (im 4-bis-6-Blattstadium) mit einer wässrigen Wirkstoffdispersion in Dosierungen von 0,06; 0,125; 0,25; 0,5; 1; 2 und 4 kg Wirksubstanz pro Hektar auf die Pflanzen gespitzt und diese bei 24°—26°C und 45—60% rel. Luftfeuchtigkeit gehalten. 15 Tage nach Behandlung wird der Versuch ausgewertet und das Ergebnis wie im pre-emergent-Versuch nach derselben Notenskala bonitiert.

Die *selektive Wirkung* erfindungsgemässer Verbindungen *gegen Avena fatua (Flughafer)* in Weizen-*kulturen* wurde durch folgenden Versuch nachgewiesen:

Im Gewächshaus wurden Töpfe mit Samen von Weizen und Flughafer angesät. Nach ca. 2 Wochen, wenn die Pflanzen aufgelaufen sind und das 3—4 Blattstadium erreich haben, werden sie mit verdünnten wässerigen Suspensionskonzentraten gespritzt. Die Wirkstoffmenge ist so gehalten, dass sie einer Aufwandmenge im Feld von 1/8, 1/4, 1/2, 1 oder 2 kg pro Hektar entspricht. Die Töpfe werden dann im Gewächshaus bei 24—26°C und 45—60% Luftfeuchtigkeit gehalten. Der Versuch wird nach 3 Wochen gemäss der oben gegebenen Notenskala ausgewertet.

Neben erfindungsgemässen Verbindungen sind folgende Substanzen geprüft worden:

A. $\alpha$ - [4 - (4' - Trifluormethylphenoxy) - phenoxy] - propionsäure - dimethylamino - äthylester, bekannt aus der DOS 2 617 804.

B. $\alpha$ - [4 - (4' - Trifluormethylphenoxy) - phenoxy] - propionsäure - 2 - (trimethylammonium) - äthylester - jodid, bekannt aus der DOS 2 617 804.

C. $\alpha$ - [4 - (3',5' - Dichlorpyridyl - 2' - oxy) - phenoxy] - propionsäure - morpholin - amid, bekannt aus der DOS 2 546 251.

D. α - [4 - (2',4' - Dichlorphenoxy) - phenoxy] - propionsäure - 2N - pyrrolidium - äthyl - ester - chlorid, bekannt aus der DOS 2 623 558.

Die Resultate sind in der untenstehenden Tabelle zusammengefasst.

| Pflanze | Flughafer (avena fatua) | | | | Weizen | | | |
|---|---|---|---|---|---|---|---|---|
| Aufwandmenge in kg/ha | 2 | 1 | 1/2 | 1/4 | 2 | 1 | 1/2 | 1/4 |
| Verbindung No. | | | | | | | | |
| 1 | 1 | 1 | 1 | 1 | 4 | 8 | 9 | 9 |
| 2 | 1 | 1 | 1 | 1 | 6 | 8 | 9 | 9 |
| 3 | 1 | 1 | 2 | 2 | 9 | 9 | 9 | 9 |
| A | 1 | 1 | 2 | 3 | 1 | 2 | 4 | 5 |
| B | 1 | 1 | 2 | 7 | 1 | 2 | 9 | 9 |

Unter diesen Versuchsbedingungen vernichten die erfindungsgemässen Verbindungen 1, 2 und 3 den Flughafer während der Weizen geschont wird.

In einem weiteren erweiterten Versuch wurden folgende Resultate erhalten:

| Pflanze | Avena fatua | | | | Lolium perenne | | | | Alopecurus myosuroides | | | | Rottboelia exaltata | | | | Digitaria sanguinalis | | | | Echinochloa crus galli | | | | Weizen | | | | Soja | | | | Zuckerrüben | | | |
|---|---|---|---|---|---|---|---|---|---|---|---|---|---|---|---|---|---|---|---|---|---|---|---|---|---|---|---|---|---|---|---|---|---|---|---|---|
| Aufwandmenge kg/ha | 1 | 1/2 | 1/4 | 1/8 | 1 | 1/2 | 1/4 | 1/8 | 1 | 1/2 | 1/4 | 1/8 | 1 | 1/2 | 1/4 | 1/8 | 1 | 1/2 | 1/4 | 1/8 | 1 | 1/2 | 1/4 | 1/8 | 1 | 1/2 | 1/4 | 1/8 | 1 | 1/2 | 1/4 | 1/8 | 1 | 1/2 | 1/4 | 1/8 |
| Verbindung | | | | | | | | | | | | | | | | | | | | | | | | | | | | | | | | | | | | |
| 1 | 1 | 1 | 1 | 3 | 1 | 2 | 2 | 3 | 1 | 2 | 2 | 2 | 1 | 1 | 1 | 1 | 1 | 1 | 1 | 1 | 1 | 1 | 1 | 1 | 8 | 9 | 9 | 9 | 9 | 9 | 9 | 9 | 9 | 9 | 9 | 9 |
| 2 | 1 | 1 | 1 | 2 | 1 | 2 | 2 | 6 | 1 | 2 | 3 | 4 | 1 | 1 | 1 | 1 | 1 | 1 | 1 | 1 | 1 | 1 | 1 | 1 | 8 | 9 | 9 | 9 | 9 | 9 | 9 | 9 | 9 | 9 | 9 | 9 |
| 3 | 1 | 2 | 2 | 3 | 2 | 3 | 7 | 8 | 3 | 4 | 4 | 9 | 1 | 1 | 2 | 4 | 1 | 2 | 2 | 2 | 1 | 1 | 1 | 1 | 9 | 9 | 9 | 9 | 9 | 9 | 9 | 9 | 9 | 9 | 9 | 9 |
| 11 | 1 | 1 | 1 | 2 | 1 | 1 | 1 | 1 | 1 | 1 | 1 | 2 | 1 | 1 | 1 | 1 | 1 | 1 | 1 | 1 | 1 | 1 | 1 | 1 | 1 | 9 | 9 | 9 | 9 | 9 | 9 | 9 | 9 | 9 | 9 | 9 |
| 14 | 1 | 1 | 1 | 2 | 1 | 1 | 1 | 3 | 1 | 1 | 1 | 2 | 1 | 1 | 1 | 1 | 1 | 1 | 1 | 1 | 1 | 1 | 1 | 1 | 3 | 9 | 9 | 9 | 9 | 9 | 9 | 9 | 9 | 9 | 9 | 9 |
| 19 | 1 | 1 | 1 | 6 | 1 | 1 | 5 | 6 | 1 | 1 | 1 | 3 | 1 | 1 | 1 | 2 | 1 | 1 | 1 | 2 | 1 | 1 | 1 | 1 | 7 | 9 | 9 | 9 | 9 | 9 | 9 | 9 | 9 | 9 | 9 | 9 |
| C | 6 | 9 | 9 | 9 | 8 | 9 | 9 | 9 | 7 | 7 | 8 | 8 | 7 | 9 | 9 | 9 | 2 | 2 | 6 | 6 | 1 | 1 | 7 | 9 | 9 | 9 | 9 | 9 | 9 | 9 | 9 | 9 | 9 | 9 | 9 | 9 |
| D | 7 | 8 | 9 | – | 9 | 9 | 9 | – | 9 | 9 | 9 | – | 7 | 8 | 9 | – | 7 | 8 | 8 | – | 1 | 1 | 2 | – | 9 | 9 | 9 | 9 | 9 | 9 | 9 | 9 | 9 | 9 | 9 | 9 |

**Patentansprüche**

1. Pyridyloxy-phenoxy-$\alpha$-propionsäure-aminoalkyl-ester der Formel I

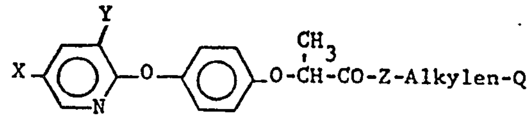

(I)

worin

X ein Halogenatom oder die Trifluormethylgruppe,

Y Wasserstoff, ein Halogenatom oder die Trifluormethylgruppe,

Z Sauerstoff oder Schwefel,

"Alkylen" einen unverzweigten oder verzweigten, gegebenenfalls durch Sauerstoff unterbrochener $C_2$—$C_6$ Alkylrest,

Q ein Amino- oder Ammonio-Rest

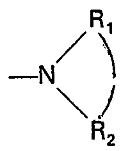 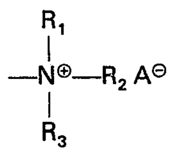 oder 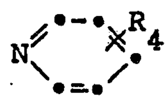

$R_1$ Wasserstoff, $C_1$—$C_6$ *Alkyl* unsubstituiert oder substituiert durch Hydroxyl, Halogen, Cyano, $C_1$—$C_4$ Alkoxy, Carboxyl oder $C_1$—$C_4$ Alkoxycarbonyl, $C_3$—$C_6$ *Alkenyl,* gegebenenfalls durch Halogen substituiert, $C_3$—$C_6$ *Alkinyl,*

$R_2$ dasselbe wie $R_1$ oder *Phenyl,* unsubstituiert oder durch Halogen, $C_1$—$C_4$ Alkyl, $C_1$—$C_4$ Alkoxy, $C_1$—$C_4$ Alkylthio, Cyano, Nitro oder Trifluormethyl substituiert,

$R_1$ und $R_2$ zusammen mit dem Stickstoffatom an das sie gebunden sind ebenfalls einen 5—6 gliedrigen Heterocyclus, der als zweites Heteroatom, Sauerstoff, Schwefel oder Stickstoff enthalten und durch $C_1$—$C_4$ Alkyl oder Phenyl substituiert sein kann,

$R_3$ dasselbe wie $R_1$

$R_4$ Wasserstoff, Methyl, Aethyl oder Halogen und

$A^{\ominus}$ das Anion einer organischen oder anorganischen Säure

bedeuten.

2. Die Pyridyloxy-phenoxy-$\alpha$-propionsäure-aminoalkyl-ester gemäss Anspruch 1, der Formel

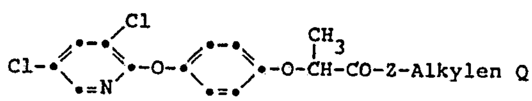

worin Z, "Alkylen" und Q die unter Formel I gegebene Bedeutung haben.

3. $\alpha$ - [4 - (3',5' - Dichlorpyridyl - 2' - oxy) - phenoxy] - propionsäure - - dimethyl - aminoäthyl - ester gemäss Anspruch 1.

4. $\alpha$ - [4 - (3',5' - Dichlorpyridyl - 2' - oxy) - phenoxy] - thiopropionsäure - s - diäethylaminoäthyl - ester gemäss Anspruch 1.

5. $\alpha$ - [4 - (3',5' - Dichlorpyridyl - 2' - oxy) - phenoxy] - propionsäure - 2 - morpholino-äthylester - iodid gemäss Anspruch 1.

6. $\alpha$ - [4 - (3',5' - Dichlorpyridyl - 2' - oxy) - phenoxy] - propionsäure - 2 - diäthylamino-äthyl - ester gemäss Anspruch 1.

7. Verfahren zur Herstellung der neuen ungesättigten Pyridyloxy-phenoxy-propionsäure-alkyl-amino-ester der Formel I des Patentanspruch 1, dadurch gekennzeichnet, dass man ein entsprechendes Propionsäure-halogenid der Formel II

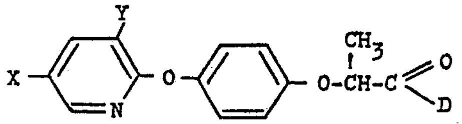

(II)

worin die X und Y die in Formel I, Anspruch 1 gegebene Bedeutung haben und D ein Halogenatom oder einen reaktionsfähigen Esterrest bedeuten, in Gegenwart eines basischen Säureakzeptors mit einem Amino = alkyl — Alkohol oder-Thiol der Formel III

H—Z-Alkylen-Q (III),

worin "Alkylen", Q und Z wie unter Formel I, Anspruch 1 definiert sind, in an sich bekannter Weise umsetzt.

8. Verfahren zur Herstellung der Pyridyloxy-phenoxy-propionsäure aminoalkyl-ester der Formel I, Patentanspruch 1, dadurch gekennzeichnet, dass man einen Hydroxyphenyl-pyridyläther oder ein Salz desselben, von der Formel IV

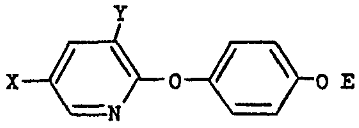

(IV)

worin X und Y die unter Formel I, Anspruch 1 gegebene Bedeutung haben und E Wasserstoff oder das Aequivalent eines Alkali- oder Erdalkalimetall-Kations darstellt, mit einem $\alpha$-Halogenpropion(thiol)-säureester der Formel V

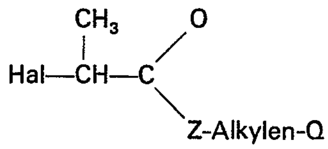

(V)

worin Hal ein Halogenatom bedeutet und "Alkylen", Q und Z die unter Formel I, Anspruch 1 gegebene Bedeutung haben in Gegenwart eines säurebindenden Mittels umsetzt.

9. Verhahren zur Herstellung von Aminoalkyl Estern von 4-(Pyridyl-2'-oxy)-$\alpha$-phenoxy-propionthiolsäuren der Formel I des Patentanspruchs 1, worin Z Schwefel ist, dadurch gekennzeichnet, dass man die genannte freie Propionthiolsäure oder ein Metallsalz derselben mit einem Aminoalkyl- Halogenid der Formel Hal-Alkylen-Q, in Gegenwart einer Base umsetzt.

10. Verfahren zur Herstellung von Pyridyloxy-phenoxy-propionsäure-ammonioalkylester der Formel I, Patentanspruch I in denen Q eine quaternäre Ammoniumgruppe bedeutet, dadurch gekennzeichnet, dass man eine Verbindung der Formel VI

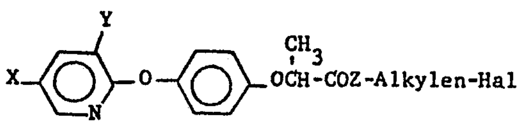

(VI)

worin "Alkylen", X Y und Z die unter Formel, Anspruch 1 gegebene Bedeutung haben, während "Hal" ein Halogenatom bedeutet, mit einem Amin der Formel VII

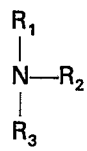

(VII)

worin $R_1$, $R_2$ und $R_3$ die unter Formel I, Patentanspruch 1 gegebene Bedeutung haben, bei Normaldruck, in einem Lösungsmittel und einer Temperatur die zwischen der Raumtemperatur und dem Siedepunkt des Lösungsmittels liegt umsetzt.

11. Verfahren gemäss Patentanspruch 10, dadurch gekennzeichnet, dass man eine Verbindung der Formel I, in der Q einen Aminorest

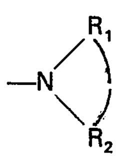

darstellt, mit einer Verbindung der Formel VIII

$A—R_3$ (VIII)

wobei $R_1$, $R_2$, $R_3$ und A die unter Formel I, Patentanspruch 1 gegebenen Bedeutungen haben, bei Normaldruck, in einem Lösungsmittel und einer Temperatur die zwischen der Raumtemperatur und dem Siedepunkt des Lösungsmittels liegt, umsetzt.

12. Herbizides Mittel, dadurch gekennzeichnet, dass es als wirksame Komponente einen Pyridyloxy-phenoxy-propionsäure-aminoalkylester der Formel I, Patentanspruch 1 enthält.

13. Die Verwendung der Pyridyloxy-phenoxy-propionsäure-aminoalkylester der Formel I, Patentanspruch 1 zur Bekämpfung von monocotylen Unkräutern.

14. Die Verwendung gemäss Patentanspruch 13, zur selektiven Bekämpfung von monocotylen Unkräutern speziell von Avena fatua in Kulturpflanzenbeständen wie z.B. Weizen.

**Claims**

1. A pyridyloxy-phenoxy-$\alpha$-propionic acid-aminoalkyl ester of the formula I

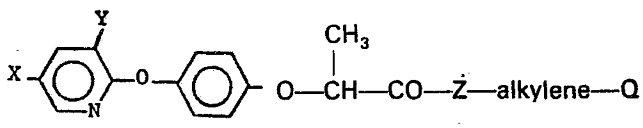

(I)

in which

X is a halogen atom or the trifluoromethyl group,
Y is hydrogen, a halogen atom or the trifluoromethyl group,
Z is oxygen or sulfur,
"alkylene" is a straight-chain or branched-chain $C_2$—$C_6$ alkyl group which can be interrupted by oxygen,
Q is an amino or ammonio group,

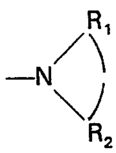 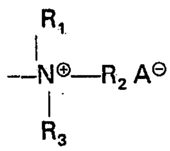 or 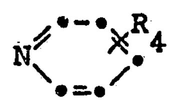

$R_1$ is hydrogen, $C_1$—$C_6$ alkyl unsubstituted or substituted by hydroxyl, halogen, cyano, $C_1$—$C_4$ alkoxy, carboxyl or $C_1$—$C_4$ alkoxycarbonyl, or it is $C_3$—$C_6$ alkenyl unsubstituted or substituted by halogen, or it is $C_3$—$C_6$ alkynyl,
$R_2$ is the same as $R_1$ or it is phenyl unsubstituted or substituted by halogen, $C_1$—$C_4$ alkyl, $C_1$—$C_4$ alkoxy, $C_1$—$C_4$ alkylthio, cyano, nitro or trifluoromethyl,
$R_1$ and $R_2$ together with the nitrogen atom to which they are bound are also a 5-6-membered heterocycle which can contain as second hetero atom oxygen, sulfur or nitrogen, and can be substituted by $C_1$—$C_4$ alkyl or phenyl,
$R_3$ is the same as $R_1$,
$R_4$ is hydrogen, methyl, ethyl or halogen, and
$A^\ominus$ is the anion of an organic or inorganic acid.

2. The pyridyloxy-phenoxy-$\alpha$-propionic acid-aminoalkyl ester according to Claim 1 of the formula

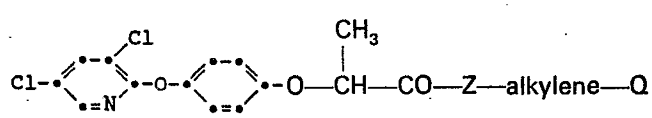

in which Z, "alkylene" and Q are as defined under the formula I.

3. $\alpha$ - [4 - (3',5' - Dichloropyridyl - 2' - oxy) - phenoxy] - propionic acid - dimethyl - aminoethyl ester according to Claim 1.

4. $\alpha$ - [4 - (3',5' - Dichloropyridyl - 2' - oxy) - phenoxy] - thiopropionic acid - s - diethylaminoethyl ester according to Claim 1.

5. $\alpha$ - [4 - (3',5' - Dichloropyridyl - 2' - oxy) - phenoxy] - propionic acid - 2 - morpholinoethyl ester iodide according to Claim 1.

6. $\alpha$ - [4 - (3',5' - Dichloropyridyl - 2' - oxy) - phenoxy] - propionic acid - 2 - diethylamino ethyl ester according to Claim 1.

7. A process for producing the novel unsaturated pyridyloxy-phenoxy-propionic acid-aklylamino ester of the formula I of Claim 1, characterised in that a corresponding propionic acid halide of the formula II

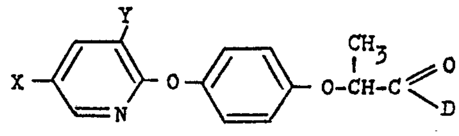

(II),

in which X and Y are as defined under the formula I, Claim 1, and D is a halogen atom or a reactive ester radical, is reacted in the presence of a basic acid acceptor, with an aminoalkyl alcohol or aminoalkyl thiol of the formula III

H—Z-alkylene-Q (III),

in which "alkylene", Q and Z are as defined under the formula I, Claim 1, in a manner known per se.

8. A process for producing the pyridyloxy-phenoxy-propionic acid-aminoalkyl ester of the formula I, Claim 1, characterised in that a hydroxyphenyl-pyridyl ether, or a salt thereof, of the formula IV

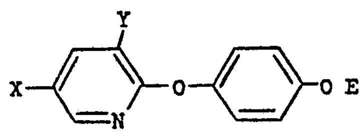

(IV),

in which X and Y are as defined under the formula I, Claim 1, and E is hydrogen or the equivalent of an alkali metal cation or alkaline-earth metal cation, is reacted with an $\alpha$-halogenopropionic(thiol)acid ester of the formula V

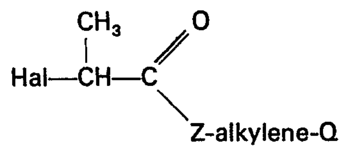

(V),

in which "Hal" is a halogen atom, and "alkylene", Q and Z are as defined under the formula I, Claim 1, in the presence of an acid-binding agent.

9. A process for producing aminoalkyl esters of a 4-(pyridyl-2′-oxy)-$\alpha$-phenoxy-thiopropionic-(thiol)acid of the formula I of Claim 1, in which Z is sulfur, characterised in that the said free thiopropionic(thiol)acid, or a metal salt thereof, is reacted with an aminoalkyl halide of the formula Hal-alkylene-Q in the presence of a base.

10. A process for producing a pyridyloxy-phenoxy-propionic acid ammonioalkyl ester of the formula I of Claim 1 in which Q is a quaternary ammonium group, characterised in that a compound of the formula VI

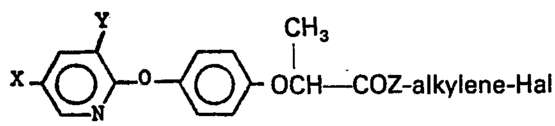

(VI),

in which "alkylene", X, Y and Z are as defined under the formula I, Claim 1, whilst "Hal" is a halogen atom, is reacted with an amine of the formula VII

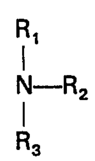

(VII),

in which $R_1$, $R_2$ and $R_3$ are as defined under the formula I, Claim 1, under normal pressure, in a solvent and at a temperature between room temperature and the boiling point of the solvent.

11. A process according to Claim 10, characterised in that a compound of the formula I, in which Q is an amino group

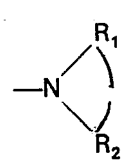

is reacted with a compound of the formula VIII

$$A—R_3 \quad (VIII),$$

in which $R_1$, $R_2$, $R_3$ and A are as defined under the formula I, Claim 1, under normal pressure, in a solvent and at a temperature between room temperature and the boiling point of the solvent.

12. A herbicidal composition containing a pyridyloxy-phenoxy-propionic acid-aminoalkyl ester of the formula I, Claim 1, as active ingredient.

13. A method of controlling monocotyledonous weeds at a locus, which method comprises applying to the said locus a pyridyloxy-phenoxy-propionic acid aminoalkyl ester of the formula I, Claim 1.

14. A method according to Claim 13 of selectively controlling monocotyledonous weeds, especially Avena fatua, in cultivated crops, for example wheat.

## Revendications

1. Aminoalcoylesters d'acide pyridyloxy-phénoxy-$\alpha$-propionique de formule I:

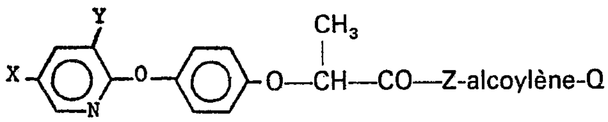

(I)

où

X représente un atome d'halogène ou le groupe trifluorométhyle,

Y représente un hydrogène, un atome d'halogène ou le groupe trifluorométhyle,

Z représente un oxygène, ou un soufre

"alcoylène" représente un radical alcoyle en $C_2$ à $C_6$ non ramifié ou ramifié, éventuellement interrompu par un oxygène,

Q représente un radical amino ou ammonio

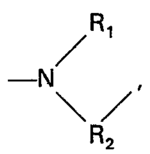, 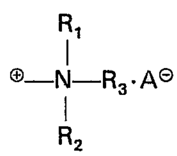 ou 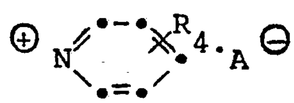

$R_1$ représente un hydrogène, un *alcoyle* en $C_1$ à $C_6$ non substitué ou substitué par un hydroxyle, un halogène, un cyano, un alcoxy en $C_1$ à $C_4$, un carboxyle ou un alcoxycarbonyle en $C_1$ à $C_4$, un *alcényle* en $C_3$ à $C_6$, éventuellement substitué par un halogène, un *alcynyle en $C_3$* à $C_6$

$R_2$ a la même signification que $R_1$ ou représente un *phényle,* non substitué ou substitué par un halogène, un alcoyle en $C_1$ à $C_4$, un alcoxy en $C_1$ à $C_4$, un alcoylthio en $C_1$ à $C_4$, un cyano, un nitro ou un trifluorométhyle,

$R_1$ et $R_2$ représentent également ensemble avec l'atome d'azote auquel ils sont liés, un hétérocycle ayant de 5 à 6 chaînons, qui peut contenir comme deuxième hétéroatome un oxygène, un soufre ou un azote et qui peut être substitué par un alcoyle en $C_1$ à $C_4$ ou un phényle,

$R_3$ a la même signification que $R_1$,

$R_4$ représente un hydrogène, un méthyle, un éthyle ou un halogène et

$A^{\ominus}$ représente l'anion d'un acide organique ou inorganique.

2. Les aminoalcoylesters d'acide pyridyloxy-phénoxy-$\alpha$-propionique selon la revendication 1, de formule

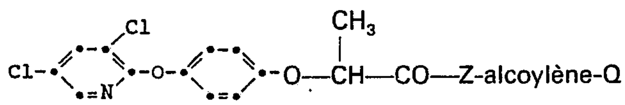

où Z, "alcoylène" et Q ont la signification donnée pour la formule I.

3. Diméthylaminoéthylester de l'acide $\alpha$-(4-(3',5'-dichloropyridyl-2'-oxy)-phénoxy)-propionique.

4. S-diéthylaminoéthylester de l'acide $\alpha$-(4-(3',5'-dichloropyridyl-2'-oxy)-phénoxy)-thiopropionique.

5. Iodure de 2-morpholinoéthylester de l'acide $\alpha$-(4-(3',5'-dichloropyridyl-2'-oxy)-phénoxy)-propionique.

6. 2-diéthylaminoéthylester de l'acide $\alpha$-(4-(3',5'-dichloropyridyl-2'-oxy)-phénoxy)-propionique

selon la revendication 1.

7. Procédé de préparation des nouveaux alcoylaminoesters insaturés d'acide pyridyloxy-phénoxy-propionique de formule I selon la revendication 1, caractérisé en ce qu'on fait réagir un halogénure d'acide propionique correspondant de formule II

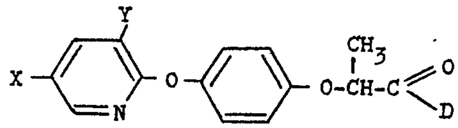

(II)

où X et Y ont la signification donnée pour la formule I et où D représente un atome d'halogène ou un radical ester réactif, en présence d'un accepteur d'acide basique avec un aminoalcoyl-alcool ou -thiol de formule III:

H—Z-alcoylène-Q (III)

où "alcoylène", Q et Z sont définis comme pour la formule I de la revendication 1.

8. Procédé de préparation des aminoalcoylesters d'acide pyridyloxy-phénoxy-propionique de formule I selon la revendication 1, caractérisé en ce qu'on fait réagir un hydroxyphénylpyridyléther ou un de ses sels, de formule IV:

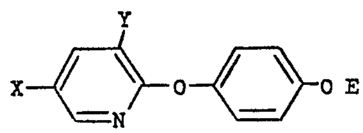

(IV)

où X et Y ont la signification donnée pour la formule I de la revendication 1 et où E représente un hydrogène ou l'équivalent d'un cation de métal alcalin ou alcalino-terreux, avec un ester d'acide $\alpha$-halogénopropionothiolique de formule V

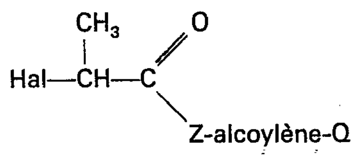

(V)

où Hal représente un atome d'halogène et "alcoylène", Q et Z ont la signification donnée pour la formule I de la revendication 1, en présence d'un agent liant acide.

9. Procédé de préparation d'aminoalcoylester d'acides 4-(pyridyl-2'-oxy)-$\alpha$-phénoxy-propionothiolique de formule I selon la revendication 1, où Z est un soufre, caractérisé en ce qu'on fait réagir l'acide propionothiolique libre mentionné ou un de ses sels métalliques avec un halogénure d'aminoalcoyle de formule Hal-alcoylène-Q, en présence d'une base.

10. Procédé de préparation d'ammonioalcoylesters d'acide pyridyloxy-phénoxy-propionique de formule I selon la revendication 1 où Q représente un groupe ammonium quaternaire, caractérisé en ce qu'on fait réagir un composé de formule VI:

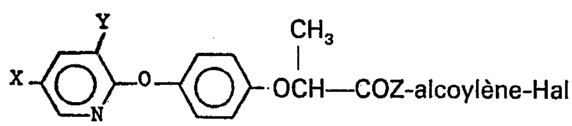

(VI)

où "alcoylène, X, Y et Z ont la signification donnée pour la formule I de la revendication 1, cependant que "Hal" représente un atome d'halogène, avec une amine de formule VII:

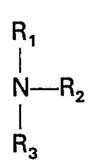

(VII)

où $R_1$, $R_2$ et $R_3$ ont la signification donnée sous la formule I de la revendication 1, à pression normale, dans un solvant et à une température qui se situe entre la température ambiante et le point d'ébullition du solvant.

11. Procédé selon la revendication 10, caractérisé en ce qu'on fait réagir un composé de formule I, où Q représente un radical amino:

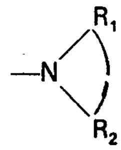

avec un composé de formule VIII:

$$A—R_3 \qquad \text{(VIII)}$$

où $R_1$, $R_2$, $R_3$ et A ont les significations données pour la formule I de la revendication 1, à pression normale, dans un solvant et à une température qui se situe entre la température ambiante et le point d'ébullition du solvant.

12. Agent herbicide, caractérisé en ce qu'il contient comme composant actif un aminoalcoylester d'acide pyridyloxy-phénoxy-propionique de formule I selon la revendication 1.

13. Application des aminoalcoylesters d'acide pyridyloxy-phénoxy-propionique de formule I selon la revendication 1 à la lutte contre les mauvaises herbes monocotylédones.

14. Application selon la revendication 13, à la lutte sélective contre les mauvaises herbes monocotylédones, en particulier contre Avena fatua dans les plantations de plantes cultivées comme par exemple le blé.